# EUROPEAN PATENT SPECIFICATION

(11) **EP 3 454 923 B1**
(45) Date of publication and mention of the grant of the patent: **13.12.2023**
(21) Application number: 17795678.6
(22) Date of filing: 09.05.2017
(51) Int. Cl.: A61M 5/168, A61M 5/172, A61M 5/14

(54) **COUNTER DEVICE**
ZÄHLERVORRICHTUNG
DISPOSITIF DE COMPTAGE

(30) Priority: 10.05.2016 FI 20165397
(43) Date of publication of application: 20.03.2019
(73) Proprietor: Monidor Oy, 90590 Oulu (FI)
(72) Inventor: SAVOLA, Mikko, 90590 Oulu (FI); POHJANVESI, Mari, 90590 Oulu (FI); PUOLITAIVAL, Seppo, 90590Oulu (FI)
(74) Representative: Kolster Oy Ab
(86) International application number: PCT/FI2017/050358
(87) International publication number: WO 2017/194836

(56) References cited:
- JP-A- 2013 226 212
- JP-A- 2013 226 212
- US-A- 4 137 940
- US-A- 4 137 940
- US-A- 4 834 744
- US-A- 4 834 744
- US-A1- 2010 309 005
- US-A1- 2010 309 005
- US-B1- 6 491 659

## Description

### Background of the invention

The invention relates to drip counters.

Drip counters are used in medicine in connection with intravenous hydration. By means of a drip counter, it is made sure that a patient acquires the desired amount of infusion liquid within the desired time.

Dripping rate of infusion liquid refers to the rate at which infusion liquid is dispensed for the patient. Conventionally, the dripping rate is indicated as the measured number of drips per minute, but the dripping rate may also be indicated as millilitres per hour, if the volume of one drip is known in advance and if the aforementioned minute-specific number of drips has been determined.

In drip counters, the measurement of the drip quantity is carried out optically by, for example, an LED (light emitting diode) light source and a phototransistor as its pair. Modern drip counter devices are such that the device measures the number of drips and, by means of its data processing unit and display, the device shows the number of the drips and the liquid volume, calculated by means of it, delivered to a patient. In addition, a drip counter features adjustable alarm thresholds by means of which the device provides an alarm for the nursing staff if the dripping rate of the infusion liquid is not at the desired level or if a preset maximum amount of infusion liquid has been administered, that is, dripped.

Prior art drip counters are limited in their structure as to what kind of a liquid chamber the separate infusion tubing to be supported to the drip counter device may have. For the above reason, the user needs to aware of the issue, and an infusion tubing with a liquid chamber suitable specifically for the counter model in question needs to be available. The problem is emphasised if the same nursing institution has drip counters using different kinds of infusion tubing, because this may confuse the persons using the devices. It is further the case that separate sets of drip tubing are typically much more expensive, which is an issue that increases the operating costs of single-use infusion tubing compared to a situation where the most cost-effective alternative could be more freely selected.

Some prior art devices are disclosed in the publications US2010309005, JP2013226212 A and EP0209659, but the structures disclosed in them do not help the solving of the aforementioned problems because the devices are not suitable for various kinds of chambers.

### Brief description of the invention

An object of the invention is thus to provide a new typ of device so as to enable the aforementioned problems to be solved or alleviated. The object of the invention is achieved by a counter device which is characterised by what is disclosed in the independent claim. Preferred embodiments of the invention are disclosed in the dependent claims.

The invention is based on modifying the structures such that the device becomes more versatile.

The advantage of the device according to the invention is its diverse applicability to different operating environments as well as its reliability and ease of use.

### Brief description of the figures

The invention will now be described in more detail in connection with preferred embodiments and with reference to the accompanying drawings, in which
Figure 1 is a front view of the device with a collarless liquid chamber,
Figure 2 is a front view of the device with a liquid chamber having a collar,
Figure 3 is a front view of the device without a liquid chamber,
Figure 4 shows the device from the back, with a liquid chamber having a collar and a latch in semi-open position,
Figure 5 shows the device from the back, with a liquid chamber having a collar and a latch in closed position,
Figure 6 shows the device from the back with a latch in closed position,
Figure 7 shows a latch part equipped with a flexible structure,
Figures 8A and 8B show the device equipped with a spring-locked latch,
Figures 9A-9E show the device at the various phases of installing a push-rotate type of latch,
Figure 10 shows locking structures of a push-rotate type of latch at its hinge side on the back surface of a branch of the device frame,
Figure 11 shows a device equipped with an indicator LED,
Figure 12 is a block diagram of the measuring function of the device,

### Detailed description of the invention

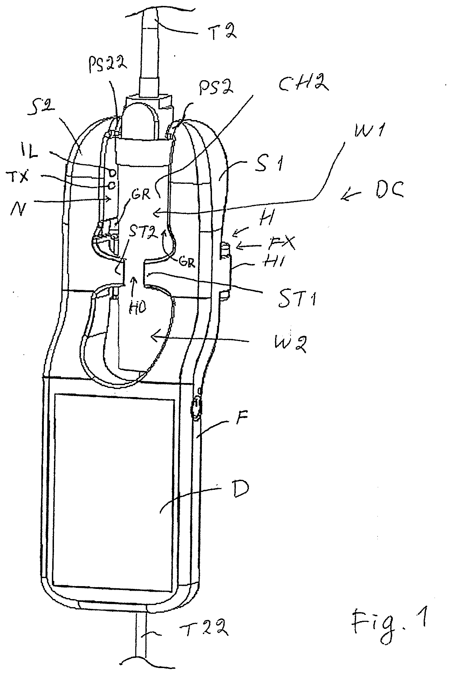

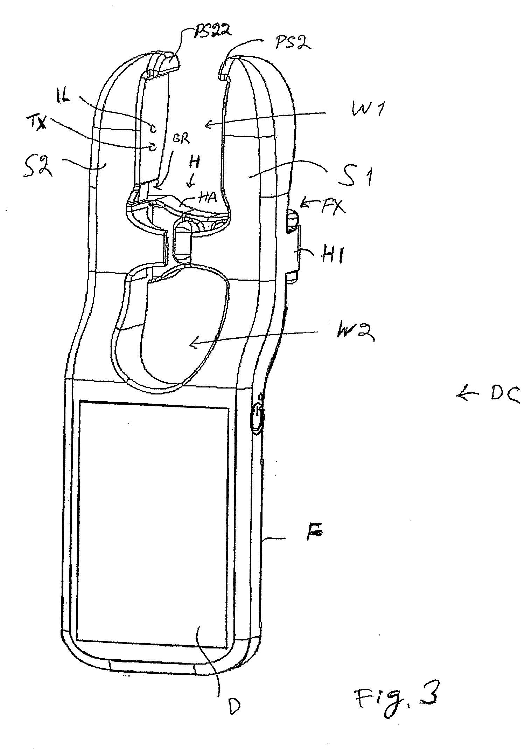

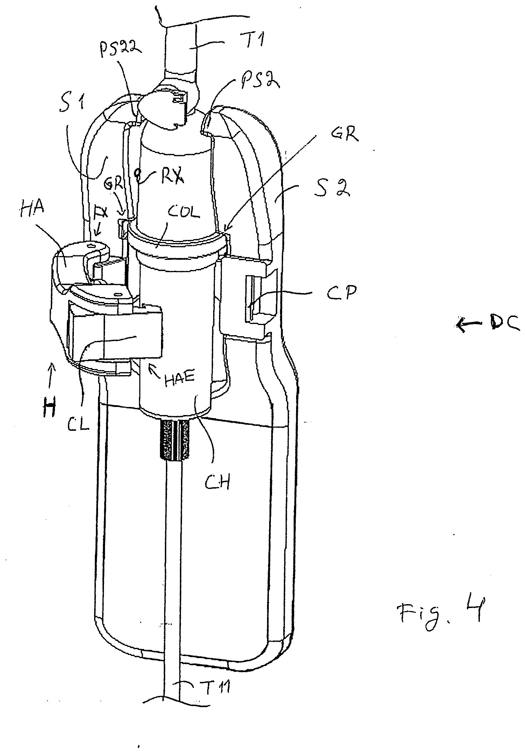

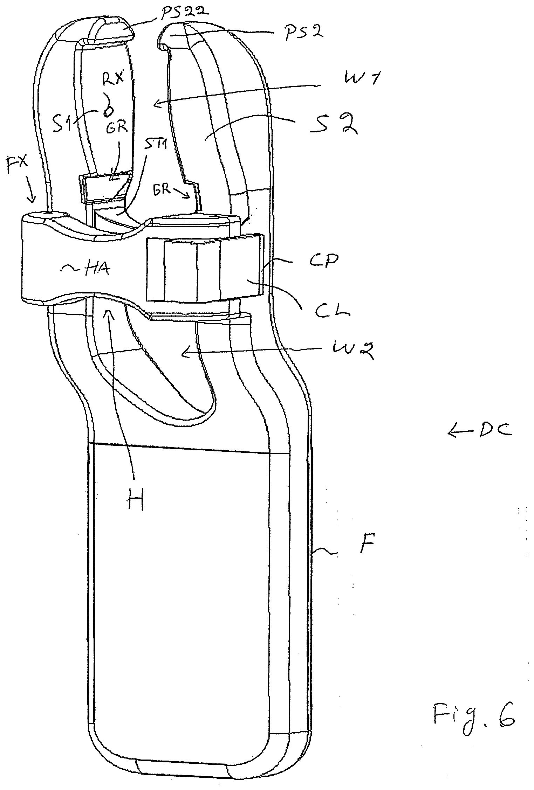

At first, the structures according to Figures 1 to 6 will be examined, in particular, which are mutually similar also as relates to the installation/fixing of the latch H, but the disclosed description is for the most part also applicable to the structures shown in the other figures, because the deviations in them mostly relate to the structures of the latch or those required by the installation of the latch.

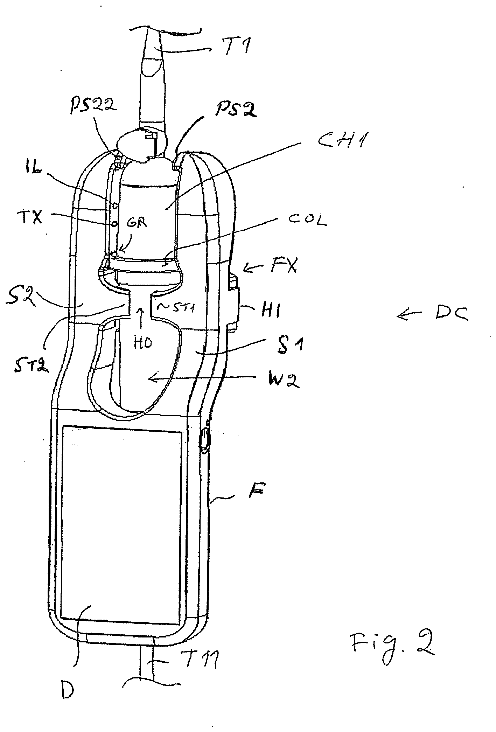

The device in question is thus a counter device DC, that is, a drip counter for counting infusion liquid drips. The liquid, in other words, the infusion liquid, may be a saline solution or Ringer solution. The invention is not limited to the type of the liquid. A medicinal substance or nutritive substances may have been added in the infusion liquid. The drips enter the infusion liquid chamber CH1, CH2 from an infusion liquid carried by a transfer channel, such as a tube T1, T2. In Figure 1, a collarless liquid chamber CH2 has been installed in the device DC, to which liquid is brought from above by the tube T2, and the tubing continues under the liquid chamber CH2 as a tube T22 which carries the liquid to a vein in a patient's body. In Figures 2, 4, and 5, a liquid chamber CH1 with a collar, that is, a collar COL has been installed in the device DC, to which liquid is brought from above by the tube T1, and the tubing continues under the liquid chamber CH1 as a tube T11 which carries the liquid to a vein in a patient's body.

The device DC comprises a frame F that has a nest N for such a separate infusion liquid chamber CH1, correspondingly for CH2. In addition, the device comprises a latch H, which is arranged to close or otherwise confine the nest N in order to keep the infusion liquid chamber CH1, CH2 in the nest N.

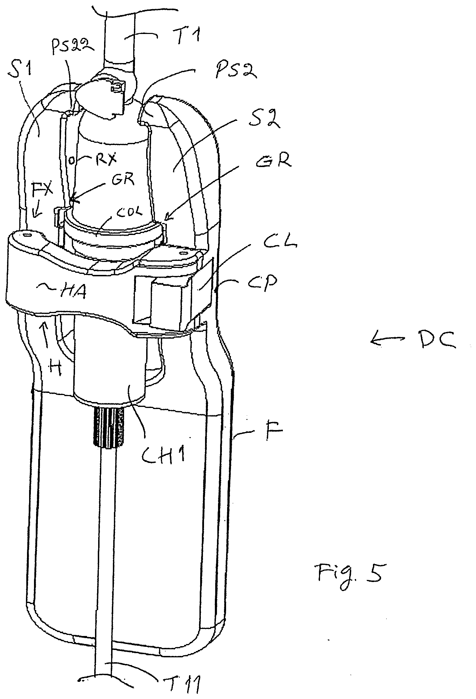

For supporting the liquid chamber CH 1provided with a collar COL, shown in Figures 2, 5 and 5, the frame F of the device DC additionally comprises, as a positioning structure GR, a positioning groove GR which positions the liquid chamber CH1 in its place in the nest N.

As regards the measurement arrangement, it is at this stage noted that the device DC comprises a measurement structure TX, RX to detect the drips, and the measurement arrangement comprises an optical measurement structure where a transmitter element TX such as a light source, such as an LED (light emitting diode), which is arranged to send a signal such as light through the liquid chamber CH1, CH2 to a receiver element RX, which may be a phototransistor, for example, that is, a transistor that measures light or another similar detector that on the basis of a change in a signal it receives detects, if a drip of liquid falls through a measurement route in the liquid chamber CH1, CH2. The LED, that is, the transmitter element TX and the optical receiver RX as its pair are placed in different branches S1, S2 of the device and their height position is such that they are higher, in other words, closer to the top of the device than the height where the latch HA is located.

With reference to the block diagram 12, the measurement arrangement comprises, in addition to the measurement structure, that is the transmitter TX and receiver RX, a microcontroller unit (MCU) in communication with the measurement structure, and a display D connected thereto. The MCU controls the operation of the device, among other things, by controlling the optical transmitter TX and by calculating, on the basis of a signal provided by the optical receiver RX, what the amount of liquid drips is per a unit of time, that is, the MCU calculates the value drips/minute. The MCU may also calculate the combined liquid volume of administered drips. The display D may at the same time be the user interface, if it is a touch screen. By means of the user interface, the user may change the settings of the device and carry out other desired measures.

The structures shown in the block diagram 12 are in the frame F of the device DC, the display D is at the bottom part of the frame F, that is, in an area below the nest N intended for the liquid chamber CH1, CH2. Instead of the visible light region, the transmitter element TX and receiver element RX may operate in the infrared (IR) region, so the transmitter element TX may be an IR-LED, for example. The latch part HA is a mere mechanical structure, because the display D and the data processing unit MCU are in the frame F and not in the part that turns in relation thereto, that is, the latch part HA, this feature increasing the reliability of operation.

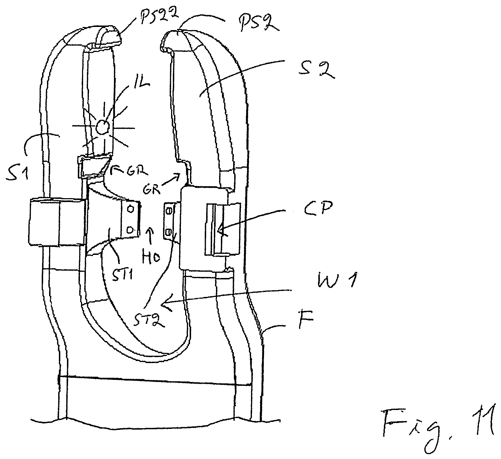

Figure 11 also shows an indicator IL, which tells the user the measurement point from the height of which the measurement of the drip from the liquid chamber CH1, CJ2 takes place. The indicator IL may be an LED of the visible light region, and the role of the indicator IL is to help the user, because the actual transmitted LED TX of the measurement is poorly detectable as regards its signal, in particular if the transmitter LED TX is an IR-LED.

Additionally or alternatively, the indicator IL may be used as follows, so the device with the help of the MCU is arranged to change the control that the indicator IL receives at its input when a liquid drip has been detected by means of the measurement structure TX, RX whereby the indicator IL is arranged to change its output signal. In practise, the situation may be one where the indicator IL controlled by the MCU is switched off or becomes substantially dimmer once a drip has been detected, this feature may act as a user's help in indicating the arrival of a drip and/or providing indicative information on the number of drips per a unit of time.

The device DC is such that for a different type of infusion liquid chamber CH2, shown in Figure 1, the frame F of the infusion device DC comprises a second positioning structure PS2, PS22, and said second positioning structure PS2, PS22 is at a distance from the positioning structure GR of the positioning groove type, being located at a different height in relation to the vertical direction of the frame F of the device DC than the positioning structure GR of the positioning groove type. In the example of the figures, the aforementioned distance is a few centimetres. The device hence comprises a second positioning structure PS2, PS22, which is arranged to prevent the rising movement of the infusion liquid chamber to the placed in the nest, in the vertical direction of the frame F. The second positioning structure PS2, PS22 arranged to prevent the rising movement of the infusion liquid chamber in the vertical direction of the frame F is thus at a distance from the positioning structure GR of the positioning groove type, being therefore located at a different height in relation to the vertical direction of the frame F than the positioning structure GR of the positioning groove type. Both of the positioning structures GR and PS2, PS22 are arranged to prevent the rising movement of the corresponding infusion liquid chamber (different chambers at different points in time) in the vertical direction of the frame F.

Said second positioning structure PS2, PS22 is in an embodiment located higher in relation to the vertical direction of the frame F, so closer to the top edge of the frame than the positioning structure GR of the positioning groove type.

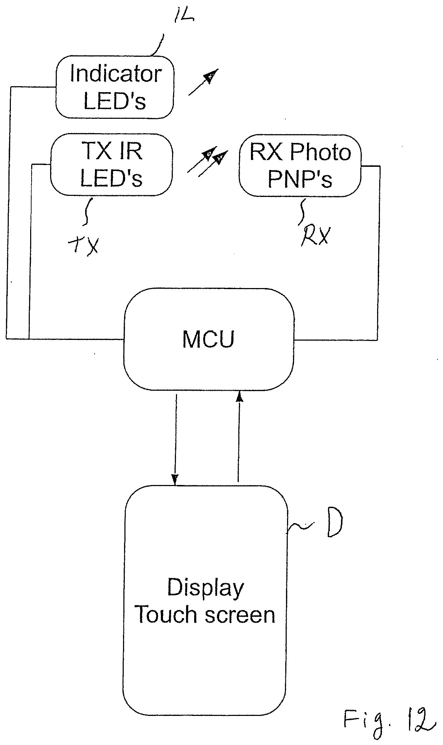

The frame F may be of plastic, such as polyethene, in which the required shapes and structures have been made at the casting phase or by machining. The frame F has placement points for the structures shown in Figure 12.

The aforementioned second positioning structure PS2, PS22 is located at the top part of the nest N comprised by the frame, in particular so that in an embodiment the second positioning structure is located at the top part of the nest N comprised by the frame at the top part of the frame F, as illustrated in the figures.

Referring to Figure 1, in particular, in more specific terms an embodiment has a structure where the second positioning structure PS2, PS22 comprises a reduction structure of the top part of the nest, arranged to prevent the rising movement of the infusion liquid chamber CH2 to be placed in the nest, in the vertical direction of the frame F.

It is discovered that the outer surface of the top part of the liquid chamber CH2 in Figure 1 acts as the counterpart for the second positioning structure PS2, PS22.

In Figures 2, 4 and 5, the collar COL outside the liquid chamber is the counterpart for the first positioning structure GR.

With reference to Figures 2, 4 and 5, it is noted that the second positioning structure PS2, PS22 in question obviously also exists on the device DC supporting a liquid chamber CH1 equipped with a collar COL, but in Figures 2, 4, and 5 that positioning structure PS2, PS22 is not in use, because a different liquid chamber CH1 has been installed in the device, which by means of its collar COL, that is, a flange, is supported to the groove GR, which, as noted, is the first positioning structure.

The physical locations of the transmitter element TX such as an LED and the receiver element RX such as a phototransistor and the indicator LED IL are best shown by simultaneously examining Figures 1 to 3, 6, and 11. It is detected that in an embodiment the transmitter element TX and the receiver element RX are placed higher than the groove GR positioning the liquid chamber CH1 provided with the collar COL, and lower than the positioning structure PS2, PS22 positioning a different kind of liquid chamber CH2 in a different usage situation. The transmitter element TX may be, for example, 8.5 mm higher than the top edge of the groove GR. The transmitter element TX may be implemented with one or more, such as three, LEDs, and if a plurality of LEDs are used, they are in an embodiment in parallel in a row.

In the figures, the transmitter element LED, so TX, is on the left branch S2 of the device when the device is as viewed from the front, so the display D side, and the receiver element RX in on the right-hand side branch S1, but the placement may also be reversed. In Figure 11, the indicator LED IL is placed on the branch S1, but in an embodiment according to Figures 1 to 3 and 6 it is preferably on the same branch S2 as the transmitter element TX.

There are good grounds to place the indicator LED IL in the vicinity of the measurement point (where the TX, RX), in an embodiment the indicator LED IL is no more than 10 mm from the measurement point, that is, 4.5 mm higher than the LED TX, for example.

Next, issues relating to the latch H will be discussed. In an embodiment, the latch is, as shown in the figures, at such a height that the latch H is at the centre area, as regards the vertical direction of the nest N, or at the area of the lower half, because in such a case the latch H makes it against the flexible, compressive surface of the liquid chamber CH1, CH2, because the centre area and bottom area of the liquid chamber CH1, CH2 are typically compressive so that the user could squeeze the liquid chamber CH1, CH2. The contact of the latch part HA of the latch H with the flexible surface of the liquid chamber CH1, CH2 establishes a simple and reliable pressure between the latch part HA of the latch H and the liquid chamber CH1, CH2.

In an embodiment, the vertical position of the latch H is such that the latch H is located closer to the bottom part of the frame F than said second positioning structure PS2, PS22. The vertical position of the latch H is such that the latch H is located closer to the bottom part of the frame F than the positioning structure GR of the positioning groove type. When both the above embodiments are taken into account, the vertical position of the latch H is thus such that the latch H is located closer to the bottom part of the frame F than either positioning structure GR, PS2/PS3. Said embodiments improve the stability of the support of the liquid chamber, that is, the liquid chamber CH1, CH2.

Referring in particular to Figures 1 to 3, in an embodiment it is the case that at the back of the nest N, the nest N, meant for the liquid chamber CH1, CH2, comprises a prevention structure ST1, ST 2 for additional support of the infusion liquid chamber, such as CH1, CH2. The prevention structure ST1, ST2 acts as the mating surface for the latch H when the latch H, HA presses the liquid chamber CH1, CH2 as in Figures 1, 2, and 5. The prevention structure ST1, ST2 is a claw structure at both edges of the nest, the tip areas of the claws, in particular, conforming with the shape of the surface of the chamber CH1, CH2 due to their curvature, and consequently centre the chamber in the right spot.

It is detected that the prevention structure ST1, ST2 comprises a transmittance area HO, which is either an opening or another transparent area that prevents visibility through the liquid chamber CH1, CH2. In the example of the figures, the prevention structure has a curved surface, the purpose of which is to conform with the surface of the liquid chamber CH1, CH2. The transmittance area HO in the prevention structure ST1, ST2 is between the different sides of the prevention structure, such as the halves ST1, ST2.

To improve the support, in an embodiment the prevention structure ST1, ST2 at the back of the nest N is substantially at the same height as the latch H that closes the nest N of otherwise confines the nest N. It is furthermore the case that in an embodiment the prevention structure ST1, ST2 is at the centre area of the nest N in relation to the vertical direction of the nest N.

In the example of the figures, the height of the prevention structure ST1, ST2 at the centre area of the transverse width of the nest N, in particular, is only approximately 1/8 of the height of the nest N. The prevention structure ST1, ST2 may also be notably higher than the aforementioned 1/8 of the nest N height, as longs as enough visibility will remain, the height of the latch of the prevention structure ST1, ST2 may be, for example, no more than one fourth or one half of the height of the nest N.

The upper part of the frame F is branch-like and comprises parallel branches S1, S2, which face each other so that the nest N for the infusion liquid chamber CH1, CH2 is between the branches S1, S2 of the frame.

For the latch H, HA not to obstruct visibility too much, in an embodiment the height of the latch H in the vertical direction of the frame is lower than the height of the nest N meant for the infusion liquid chamber CH1, CH2. The latch H is at the centre area of the height direction of the nest N so that transparency will remain both at W1 below the latch H and at W2 above the latch.

In the example of the figures, the height of the latch H, HA at the centre area of the transverse width of the nest N, in particular, is only approximately 1/5 of the height of the nest N. The latch H, HA may also be notably higher than the aforementioned 1/5 of the nest N height, as long as enough visibility will remain, the height of the latch H may be, for example, no more than one half of the height of the nest N.

In an embodiment, the nest N at the area above the latch H comprises an opening or another transparent area W1, and correspondingly at the area below the latch H, the nest N comprises an opening or another transparent area. If it has an opening, this creates the additional benefit that the user is able to squeeze, grasp, or otherwise handle the liquid chamber CH1, CH2 more easily. Instead of an opening in the areas W1, W2, there may be a transparent wall which also allows visibility.

The latch H comprises an openable, that is, turnable, that is, rotatable latch part HA and additionally a fastening FX enabling the rotation for the turning motion, that is, rotating motion of the latch part HA in relation to the frame F, and the latch H additionally comprises a closing means CL for closing the free end HAE of the latch part HA in relation to the frame F by means of a counterpart CP on the branch S2 of the frame. The rotating fastening is on the side of the branch S1 of the frame.

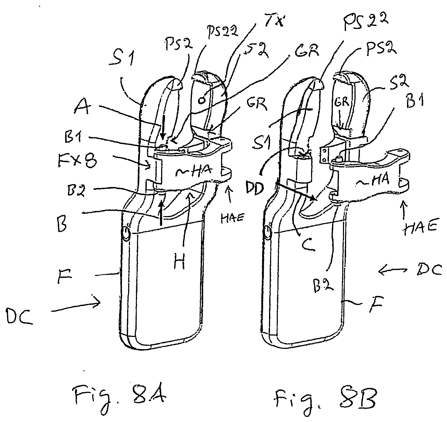

Next, the embodiment according to Figures 8A and 8B will be examined. There, the aforementioned rotating fastening, now denoted by FX8 for the latch part HA is
a quick-release rotating fastening in order to detach the latch part HA from the frame F and to attach it to the frame F. Detaching/attaching is in relation to the branch S1.

The quick-release rotating fastening FX8 of Figures 8A and 8B comprises spring buttons B1, B2 which, when pressed towards each other according to arrows A, B, causes the vertical spring pin DD in the hinge bracket HI of the frame F branch S1 to be pressed shorter, whereby the latch part HA is released from the branch S1 of the frame F as shown by arrow C.

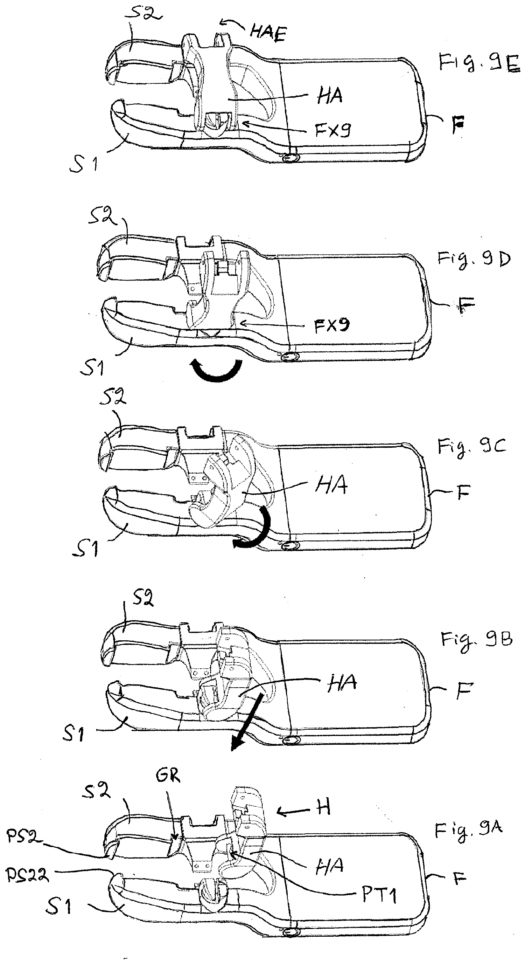

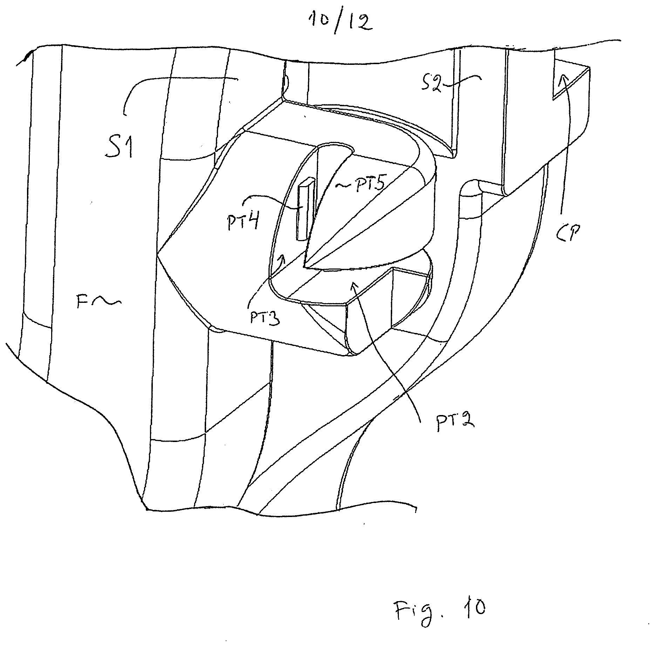

Next, and alternative for the quick-release rotating fastening is examined, now denoted with FX9, so in practise for the fastening of the latch part HA, now according to Figures 9A to 9E, and 10. The rotating fastening, now denoted with FX9, for the latch parts HA is a quick-release rotating fastening in order to detach the latch part HA from the frame F and to attach it to the frame F. Detaching/attaching is in relation to the branch S1. In this embodiment, this quick-release rotating fastening for the latch part comprises a push-rotation structure PT1, PT2, PT3, PT4, PT5 by means of which the latch part HA is arranged to be attachable to the frame F of the device and detachable from the frame F of the device.

In Figures 9A to 9E and 10, the quick-release rotating fastening FX9 for the latch part HA comprises, in the latch part HA, a pin or a similar protrusion PT1 and for the protrusion PT1 in question there are, in the frame F, that is, in particular in the branch S1 of the frame, grooves or other similar motion spaces PT2, PT3, which grooves PT2, PT3 extend to mutually intersecting directions, because this relates to the successive combination of the pushing motion and rotating motion of the latch part HA. In addition, there is a locking bulge PT4, which may be in the groove PT3 further aback. In addition, the quick-release rotating fastening FX9 comprises a wall PT5, which prevents the breaking-free motion of the protrusion PT1 of the latch part. The wall PT5 at the same time confines the groove PT3 behind it, and also confines the groove PT2 below it.

So, the latch part HA is pushed in the position according to Figures 9A, 9B towards the branch S1 of the frame F, whereby the protrusion PT1 at the end of the latch part HA proceeds along the first groove PT2 of the frame F and next, as in Figures 9C, 9D, the latch part HA is rotated clockwise along the groove PT3 further aback until the protrusion PT1 at the end of the latch part HA passes the locking bulge PT4 and locks itself behind it. This way, the latch part is quick-release locked on the brach S1 of the frame H. The detachment of the latch part HA takes place with a reverse movement. The easy detachability/attachability of the latch part HA facilitates washing the device parts.

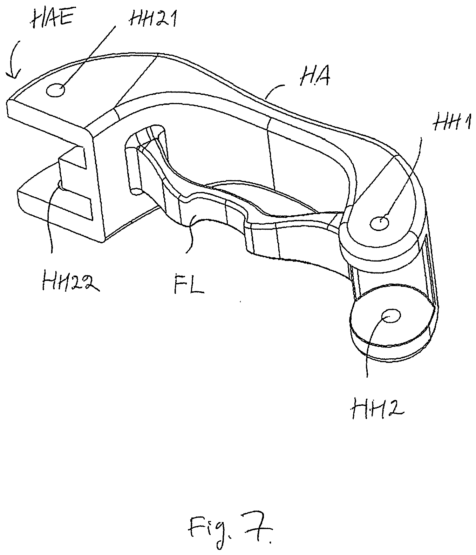

With reference to Figure 7, in an embodiment the most essential part of the latch H, that is, the latch part HA, has on its inner surface (the one against the liquid chamber CH1, CH2) a flexible structure FL, which is a flexible isthmus, for example, as in Figure 7. So, the flexible structure FL is directed towards the nest N intended for the liquid chamber CH1, CH2. Figure 7 also shows the fastening points HH1, HH2 of the latch part HA, used to fasten the latch part HA rotatably to the hinge bracket HI on the branch S1 of the frame F, and additionally are shown the fastening points HH21, HH22, to which the closing means CL comprised by the free end HAE of the latch part HA is fastened. Figures 8A, 8B or 9A to 9E do not show the closing means CL in question at the free end HAE of the latch part HA.

In an embodiment, the device is so implemented that the mechanisms and the attachment of the drip chamber, such as CH1 and the tubing, to the device, and/or their detachment, are arranged to be managed using one hand. Thus, the latch HA is a single-hand type of latch. Therefore, in an exemplary usage situation, the right hand is used to hold the device at one point so that the display D of the device is at the bottom part and the branches S1, S2 of the device point upward and the index finger touches/extends over the latch HA. By bending the finger towards the palm, the latch HA opens. Then, the drip chamber CH1, for example, provided with tubes is installed in place in the nest N and the index finger is next used to release the latch HA, and the locking is pressed in the closed position. The same principle applies to the other kind of drip chamber CH2.

A person skilled in the art will find it obvious that, as technology advances, the basic idea of the invention may be implemented in many different ways. The invention and its embodiments are thus not restricted to the above-described examples but may vary within the scope of the claims.

## Claims

1. A counter device for counting infusion liquid drips, the device comprising a frame (F) which has a nest (N) for a separate infusion liquid chamber, and a latch (H,HA) which is arranged to close or otherwise confine the nest (N) to keep the infusion liquid chamber in the nest (N), and the device frame (F) further comprising a positioning groove as a positioning structure (GR) for supporting the infusion liquid chamber, and the device also comprising a measuring structure (TX, RX, MCU) to detect the drips, wherein, for a different type of infusion liquid chamber, the frame of the infusion device comprises a second positioning structure (PS2, PS22) which is arranged to prevent the rising movement of the infusion liquid chamber to the placed in the nest, in the vertical direction of the frame (F), and said second positioning structure (PS2, PS22) arranged to prevent the rising movement of the infusion liquid chamber in the vertical direction of the frame (F) is at a distance from the positioning structure (GR) of the positioning groove type, being located at a different height in relation to the vertical direction of the frame (F) than the positioning structure (GR) of the positioning groove type.

2. A device as claimed in claim 1, **characterised in that** said second positioning structure (PSS2, PS22) is located higher in relation to the vertical direction of the frame, so closer to the top edge of the frame (F) than the positioning structure (GR) of the positioning groove type.

3. A device as claimed in claim 1 or 2, **characterised in that** said second positioning structure (PS2, PS22) is located at the top part of the nest (N) comprised by the frame(F).

4. A device as claimed in claim 3, **characterised in that** said second positioning structure (PS2, PS22) is located at the top part of the nest (N) comprised by the frame (F) at the top part of the frame.

5. A device as claimed in any one of the preceding claims, **characterised in that** said second positioning structure comprises a reduction structure of the top part of the nest, arranged to prevent the rising movement of the infusion liquid chamber placed in the nest, in the vertical direction of the frame.

6. A device as claimed in claim 1, **characterised in that** the vertical position of the latch is such that the latch is located closer to the bottom part of the frame than said second positioning structure.

7. A device as claimed in claim 1 or 5, **characterised in that** the vertical position of the latch is such that the latch is located closer to the bottom part of the frame than the positioning structure of the positioning groove type.

8. A device as claimed in claim 6 and 7, **characterised in that** the vertical position of the latch is such that the latch is located closer to the bottom part of the frame than either positioning structure.

9. A device as claimed in claim 1, **characterised in that** at the back of the nest, the nest N comprises a prevention structure (ST1, ST2) for additional support of the infusion liquid chamber (CH1, CH2).

10. A device as claimed in claim 9, **characterised in that** the prevention structure (ST1, ST2) in the nest (N) is substantially at the same height as the latch (H, HA) that closes the nest of otherwise confines the nest.

11. A device as claimed in claim 9 or 10, **characterised in that** the prevention structure (ST1, ST2) is at the centre area of the nest (N) in relation to the vertical direction of the nest (N).

12. A device as claimed in any one of the preceding claims 9 to 11, **characterised in that** the frame (F) comprises parallel branches (S1, S2), which face each other so that the nest (N) for the infusion liquid chamber is between the branches (S1, S2) of the frame.

13. A device as claimed in claim 1 or 7, **characterised in that** the height of the latch in the vertical direction of the frame is lower than the height of the nest intended for the infusion liquid chamber.

14. A device as claimed in claim 1 or 13, **characterised in that** at the area above the latch (H) the nest (N) comprises an opening or another transparent area (W1).

15. A device as claimed in claim 1, 13 or 14, **characterised in that** at the area below the latch (H) the nest (N) comprises an opening or another transparent area (W2).

16. A device as claimed in claim 1 or 7, **characterised in that** the latch (H) comprises an openable latch part (HA) and additionally a fastening enabling the rotation for the rotating motion of the latch part in relation to the frame, and a closing means (CL) for closing the free end (HAE) of the latch part (HA) in relation to the frame (F).

17. A device as claimed in claim 12 or 16, **characterised in that** the rotating fastening is at the first branch (S1) of the frame (F) and the closing means is at the second branch (S2) of the frame (F).

## Patentansprüche

1. Zählervorrichtung zum Zählen von Infusionsflüssigkeitstropfen, wobei die Vorrichtung einen Rahmen (F), der ein Nest (N) für eine separate Infusionsflüssigkeitskammer aufweist, und einen Riegel (H, HA) umfasst, der angeordnet ist, das Nest (N) zu schließen oder auf andere Weise abzugrenzen, um die Infusionsflüssigkeitskammer im Nest (N) zu halten, und wobei der Vorrichtungsrahmen (F) ferner eine Positionierungsnut als eine Positionierungsstruktur (GR) zum Stützen der Infusionsflüssigkeitskammer umfasst, und wobei die Vorrichtung außerdem eine Messstruktur (TX, RX, MCU) zum Detektieren von Tropfen umfasst, wobei,
der Rahmen der Infusionsvorrichtung für eine andere Art einer Infusionsflüssigkeitskammer eine zweite Positionierungsstruktur (PS2, PS22) umfasst, die angeordnet ist, die steigende Bewegung der im Nest platzierten Infusionsflüssigkeitskammer in die vertikale Richtung des Rahmens (F) zu verhindern, und sich die zweite Positionierungsstruktur (PS2, PS22), die angeordnet ist, die steigende Bewegung der Infusionsflüssigkeitskammer in die vertikale Richtung des Rahmens (F) zu verhindern, einen Abstand von der Positionierungsstruktur (GR) der Positionierungsnutart aufweist, und sich mit Bezug auf die vertikale Richtung des Rahmens (F) in einer anderen Höhe befindet als die Positionierungsstruktur (GR) der Positionierungsnutart.

2. Vorrichtung nach Anspruch 1, **dadurch gekennzeichnet, dass** sich die zweite Positionierungsstruktur (PS2, PS22) mit Bezug auf die vertikale Richtung des Rahmens an einer höheren Stelle befindet und somit näher an der Oberkante des Rahmens (F) als die Positionierungsstruktur (GR) der Positionierungsnutart.

3. Vorrichtung nach Anspruch 1 oder 2, **dadurch gekennzeichnet, dass** sich die zweite Positionierungsstruktur (PS2, PS22) im oberen Teil des im Rahmen (F) umfassten Nests (N) befindet.

4. Vorrichtung nach Anspruch 3, **dadurch gekennzeichnet, dass** sich die zweite Positionierungsstruktur (PS2, PS22) im oberen Teil des im Rahmen (F) umfassten Nests (N) im oberen Teil des Rahmens befindet.

5. Vorrichtung nach einem der vorhergehenden Ansprüche, **dadurch gekennzeichnet, dass** die zweite Positionierungsstruktur eine Reduzierungsstruktur des oberen Teils des Nests umfasst, die angeordnet ist, die steigende Bewegung der im Nest platzierten Infusionsflüssigkeitskammer in die vertikale Richtung des Rahmens zu verhindern.

6. Vorrichtung nach Anspruch 1, **dadurch gekennzeichnet, dass** die vertikale Position des Riegels derart ist, dass sich der Riegel näher am unteren Teil des Rahmens befindet als die zweite Positionierungsstruktur.

7. Vorrichtung nach Anspruch 1 oder 5, **dadurch gekennzeichnet, dass** die vertikale Position des Riegels derart ist, dass sich der Riegel näher am unteren Teil des Rahmens befindet als die Positionierungsstruktur der Positionierungsnutart.

8. Vorrichtung nach der Ansprüche 6 und 7, **dadurch gekennzeichnet, dass** die vertikale Position des Riegels derart ist, dass sich der Riegel näher am unteren Teil des Rahmens befindet als eine der Positionierungsstrukturen.

9. Vorrichtung nach Anspruch 1, **dadurch gekennzeichnet, dass** das Nest (N) zum zusätzlichen Stützen der Infusionsflüssigkeitskammer (CH1, CH2) hinten am Nest eine Verhinderungsstruktur (ST1, ST2) umfasst.

10. Vorrichtung nach Anspruch 9, **dadurch gekennzeichnet, dass** sich die Verhinderungsstruktur (ST1, ST2) im Nest (N) im Wesentlichen in derselben Höhe befindet wie der Riegel (H, HA), der das Nest schließt oder das Nest von andere Weise abgrenzt.

11. Vorrichtung nach Anspruch 9 oder 10, **dadurch gekennzeichnet, dass** sich die Verhinderungsstruktur (ST1, ST2) mit Bezug auf die vertikale Richtung des Nests (N) im Mittenbereich des Nests (N) befindet.

12. Vorrichtung nach einem der vorhergehenden Ansprüche 9 bis 11, **dadurch gekennzeichnet, dass** der Rahmen (F) parallele Abzweigungen (S1, S2) umfasst, die einander derart zugewandt sind, dass sich das Nest (N) für die Infusionsflüssigkeitskammer zwischen den Abzweigungen (S1, S2) des Rahmens befindet.

13. Vorrichtung nach Anspruch 1 oder 7, **dadurch gekennzeichnet, dass** die Höhe des Riegels in der vertikalen Richtung des Rahmens tiefer liegt als die Höhe des für die Infusionsflüssigkeitskammer vorgesehenen Nests.

14. Vorrichtung nach Anspruch 1 oder 13, **dadurch gekennzeichnet, dass** das Nest (N) im Bereich über dem Riegel (H) eine Öffnung oder einen anderen transparenten Bereich (W1) umfasst.

15. Vorrichtung nach Anspruch 1, 13 oder 14, **dadurch gekennzeichnet, dass** das Nest (N) im Bereich unter dem Riegel (H) eine Öffnung oder einen anderen transparenten Bereich (W2) umfasst.

16. Vorrichtung nach Anspruch 1 oder 7, **dadurch gekennzeichnet, dass** der Riegel (H) einen öffenbaren Riegelteil (HA) und zusätzlich eine Befestigung, die die Drehung für die Drehbewegung des Riegelteils mit Bezug auf den Rahmen ermöglicht, sowie ein Schließmittel (CL) zum Schließen des freien Endes (HAE) des Riegelteils (HA) mit Bezug auf den Rahmen (F) umfasst.

17. Vorrichtung nach Anspruch 12 oder 16, **dadurch gekennzeichnet, dass** sich die sich drehende Befestigung an der ersten Abzweigung (S1) des Rahmens (F) befindet und das Schließmittel sich an der zweiten Abzweigung (S2) des Rahmens (F) befindet.

## Revendications

1. Dispositif de comptage pour compter des gouttes de liquide de perfusion, le dispositif comprenant un bâti (F) qui a un nid (N) pour une chambre de liquide de perfusion séparée, et un verrou (H, HA) qui est agencé pour fermer ou sinon pour confiner le nid (N) afin de maintenir la chambre de liquide de perfusion dans le nid (N), et le bâti de dispositif (F) comprenant en outre une rainure de positionnement en tant que structure de positionnement (GR) pour supporter la chambre de liquide de perfusion, et le dispositif comprenant également une structure de mesure (TX, RX, MCU) pour détecter les gouttes, dans lequel :
pour un type différent de chambre de liquide de perfusion, le bâti du dispositif de perfusion comprend une seconde structure de positionnement (PS2, PS22) qui est agencée pour empêcher le mouvement ascendant de la chambre de liquide de perfusion placée dans le nid, dans la direction verticale du bâti (F), et ladite seconde structure de positionnement (PS2, PS22) agencée pour empêcher le mouvement ascendant de la chambre de liquide de perfusion dans la direction verticale du bâti (F) est à une distance de la structure de positionnement (GR) du type rainure de positionnement, étant positionnée à une hauteur différente par rapport à la direction verticale du bâti (F) que la structure de positionnement (GR) du type rainure de positionnement.

2. Dispositif selon la revendication 1, **caractérisé en ce que** ladite seconde structure de positionnement (PS2, PS22) est positionnée plus haut par rapport à la direction verticale du bâti, donc plus près du bord supérieur du bâti (F), que la structure de positionnement (GR) du type rainure de positionnement.

3. Dispositif selon la revendication 1 ou 2, **caractérisé en ce que** ladite seconde structure de positionnement (PS2, PS22) est positionnée au niveau de la partie supérieure du nid (N) composé par le bâti (F).

4. Dispositif selon la revendication 3, **caractérisé en ce que** ladite seconde structure de positionnement (PS2, PS22) est positionnée au niveau de la partie supérieure du nid (N) composé par le bâti (F) au niveau de la partie supérieure du bâti.

5. Dispositif selon l'une quelconque des revendications précédentes, **caractérisé en ce que** ladite seconde structure de positionnement comprend une structure de réduction de la partie supérieure du nid, agencée pour empêcher le mouvement ascendant de la chambre de liquide de perfusion placée dans le nid, dans la direction verticale du bâti.

6. Dispositif selon la revendication 1, **caractérisé en ce que** la position verticale du verrou est telle que le verrou est positionné plus à proximité de la partie inférieure du bâti que ladite seconde structure de positionnement.

7. Dispositif selon la revendication 1 ou 5, **caractérisé en ce que** la position verticale du verrou est telle que le verrou est positionné plus à proximité de la partie inférieure du bâti que la structure de positionnement du type rainure de positionnement.

8. Dispositif selon les revendications 6 et 7, **caractérisé en ce que** la position verticale du verrou est telle que le verrou est positionné plus à proximité de la partie inférieure du bâti que la structure de positionnement.

9. Dispositif selon la revendication 1, **caractérisé en ce qu'**à l'arrière du nid, le nid (N) comprend une structure de prévention (ST1, ST2) pour le support supplémentaire de la chambre de liquide de perfusion (CH1, CH2).

10. Dispositif selon la revendication 9, **caractérisé en ce que** la structure de prévention (ST1, ST2) dans le nid (N) est sensiblement à la même hauteur que le verrou (H, HA) qui ferme le nid ou bien confine le nid.

11. Dispositif selon la revendication 9 ou 10, **caractérisé en ce que** la structure de prévention (ST1, ST2) est au niveau de la zone centrale du nid (N) par rapport à la direction verticale du nid (N).

12. Dispositif selon l'une quelconque des revendications 9 à 11, **caractérisé en ce que** le bâti (F) comprend des branches (S1, S2) parallèles qui se font face de sorte que le nid (N) pour la chambre de liquide de perfusion est entre les branches (S1, S2) du bâti.

13. Dispositif selon la revendication 1 ou 7, **caractérisé en ce que** la hauteur du verrou dans la direction verticale du bâti est inférieure à la hauteur du nid prévu pour la chambre de liquide de perfusion.

14. Dispositif selon la revendication 1 ou 13, **caractérisé en ce qu'**au niveau de la zone au-dessus du verrou (H), le nid (N) comprend une ouverture ou une autre zone transparente (W1).

15. Dispositif selon la revendication 1, 13 ou 14, **caractérisé en ce qu'**au niveau de la zone au-dessous du verrou (H), le nid (N) comprend une ouverture ou une autre zone transparente (W2).

16. Dispositif selon la revendication 1 ou 7, **caractérisé en ce que** le verrou (H) comprend une partie de verrou pouvant s'ouvrir (HA) et de plus une fixation permettant la rotation pour le mouvement de rotation de la partie de verrou par rapport au bâti, et un moyen de fermeture (CL) pour fermer l'extrémité libre (HAE) de la partie de verrou (HA) par rapport au bâti (F).

17. Dispositif selon la revendication 12 ou 16, **caractérisé en ce que** la fixation rotative est au niveau de la première branche (S1) du bâti (F) et le moyen de fermeture est au niveau de la seconde branche (S2) du bâti (F).
